# EUROPEAN PATENT APPLICATION

(11) **EP 4 223 370 A1**
(43) Date of publication of application: **09.08.2023**
(21) Application number: 21875870.4
(22) Date of filing: 01.10.2021
(51) Int. Cl.: A61P 43/00, A61P 17/00, A61P 17/16, A61Q 19/00, A61K 9/12, A61K 8/02, A61K 8/19, A61K 8/46, A61K 8/68, A61K 8/73, A61K 8/81, A61K 47/08, A61K 47/18, A61K 47/20, A61K 47/32, A61K 47/38, A61K 31/164

(54) **CARBONATED AEROSOL FOR TOPICAL USE**

(30) Priority: 02.10.2020 JP 2020167994
(71) Applicant: Kao Corporation, Chuo-ku Tokyo 103-8210 (JP)
(72) Inventor: MIYAZAKI, Tadashi, Tokyo 131-8501 (JP); MATSUOKA, Megumi, Tokyo 131-8501 (JP); HARADA, Ryoko, Tokyo 131-8501 (JP); SUZUKI, Atsuhito, Tokyo 131-8501 (JP)
(74) Representative: Hoffmann Eitle
(86) International application number: PCT/JP2021/036411
(87) International publication number: WO 2022/071574

(57) **Abstract**

A carbonated aerosol external preparation for the skin, including a stock solution including the following components (A) to (D) and a propellant including the following component (E): (A) ceramide lipid; (B) an ionic surfactant; (C) a water-soluble polymer; (D) water; (E) carbon dioxide gas.

## Description

### Field of the Invention

The present invention relates to a carbonated aerosol external preparation for the skin.

### Background of the Invention

The stratum corneum is the outermost layer of the epidermis and prevents entrance of external substances from the outside and prevents moisture evaporation from the inside of the skin. The stratum corneum itself retains moisture and thus has a function to keep the skin soft and look smooth. Lipid called as intercorneocyte lipid exists in the gap between corneocytes constituting the stratum corneum so as to fill the gap between corneocytes.

Approximately 50% of the lipid composition of intercorneocyte lipid is ceramide lipid, and reduction of ceramide lipid is known to cause unfavorable conditions of the skin, including rough skin, dry skin and skin aging. Thus, externally supplementing ceramide lipid is considered to improve reduced function of the stratum corneum.

However, the problem is that since ceramide lipid is highly crystalline and has high melting point, stabilizing ceramide lipid in a preparation is difficult. Then, for stable mixing of ceramide lipid in an external preparation for the skin, combination with a specific surfactant or water-soluble polymer has been proposed (see, for example, Patent Literatures 1, 2).

Carbon dioxide gas is known to have effects of promoting blood circulation and hyperthermia effect, and aerosol type cosmetic using carbon dioxide gas as a propellant has been used. For example, Patent Literature 3 discloses an aerosol cosmetic with excellent skin penetration, comprising carbon dioxide gas as a propellant, solid fat, powder having a specific particle size and a nonionic surfactant.

### Patent Literatures

Patent Literature 1: JP-A-2008-81407
Patent Literature 2: JP-A-2012-214469
Patent Literature 3: JP-A-2018-43932

### Summary of the Invention

The present invention provides a carbonated aerosol external preparation for the skin, comprising a stock solution comprising the following components (A) to (D) and a propellant comprising a component (E):
(A) ceramide lipid
(B) an ionic surfactant
(C) a water-soluble polymer
(D) water
(E) carbon dioxide gas

### Detailed Description of the Invention

The present inventors considered an aerosol external preparation for the skin, comprising ceramide lipid and using carbon dioxide gas as a propellant, however, they found that emulsion stability of an emulsion containing ceramide lipid is reduced when carbon dioxide gas coexists, and the problem is that ceramide lipid is easily precipitated at high temperature, so that spraying from the aerosol container becomes impossible.

The present inventors found that an aerosol external preparation for the skin with excellent emulsion stability and excellent moisture retention properties and applicability can be prepared by using carbon dioxide gas as a propellant and combining ceramide lipid with an ionic surfactant and a water-soluble polymer.

The carbonated aerosol external preparation for the skin of the present invention can stably emulsify ceramide lipid and has excellent moisture retention properties and applicability.

### • Stock solution

The stock solution in the carbonated aerosol external preparation for the skin of the present invention comprises the following components (A) to (D).

### [Component (A): Ceramide lipid]

The ceramide lipid of the component (A) may be any one of natural ceramide and pseudo-ceramide. It is preferable that the component (A) contains one or more selected from a ceramide lipid of the following formula (1) or (2).

Natural ceramide is represented by the formula (1) and may be naturally derived ceramide or a synthesized product having the same structure. 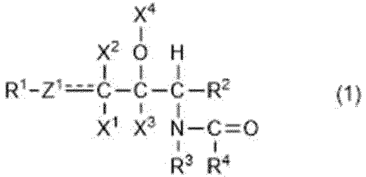 [in which R¹ represents a linear, branched or cyclic saturated or unsaturated hydrocarbon group having 7 to 19 carbon atoms, which is optionally substituted with a hydroxyl group; Z¹ represents a methylene group or a methine group; X¹, X² and X³ are each independently a hydrogen atom, a hydroxyl group or an acetoxy group; X⁴ represents a hydrogen atom or forms an oxo group together with an adjacent oxygen atom (when Z¹ is a methine group, one of X¹ and X² is a hydrogen atom, and the other is not present, and when X⁴ forms an oxo group, X³ is not present); R² represents a hydroxymethyl group or an acetoxymethyl group; R³ represents a hydrogen atom or an alkyl group having 1 to 4 carbon atoms; R⁴ represents a linear, branched or cyclic saturated or unsaturated hydrocarbon group having 5 to 30 carbon atoms, which is optionally substituted with a hydroxyl group, or a group in which a linear or branched saturated or unsaturated fatty acid having 8 to 22 carbon atoms, which is optionally substituted with a hydroxyl group, is ester-bonded to the ω terminal of the alkyl group; and the dotted line means that the bond may be an unsaturated bond.]

Examples thereof include a compound in which, in the formula (1), R¹ is a linear alkyl group having preferably 7 to 19, more preferably 13 to 15 carbon atoms; R⁴ is a linear alkyl group having 9 to 27 carbon atoms, which is optionally substituted with a hydroxyl group, or a linear alkyl group having 9 to 27 carbon atoms, to which linoleic acid is ester-bonded. Furthermore, it is preferable that X⁴ represents a hydrogen atom or forms an oxo group together with an oxygen atom. In particular, tricosyl, 1-hydroxypentadecyl, 1-hydroxytricosyl, heptadecyl, 1-hydroxyundecyl and a nonacosyl group in which linoleic acid is ester-bonded at the ω position are preferred as R⁴.

One or more selected from sphingosine, dihydrosphingosine, phytosphingosine and ceramide Type 1 to 7 prepared by amidation of sphingadienine (e.g., pig's and human ceramides shown in Fig. 2 of J. Lipid Res., 24: 759 (1983) and Fig. 4 of J. Lipid. Res., 35: 2069 (1994)) are preferred as natural ceramide. Examples also include an N-alkyl form (an N-methyl form) thereof.

As the natural ceramide, a natural optically active form (D(-) form), a non-natural optically active form (L(+) form) or a mixture of a natural form and a non-natural form may also be used. The relative configuration of the above compounds may be a configuration of the natural form, a configuration of the non-natural form other than that, or a mixture thereof. In particular, one or more selected from compounds such as CERAMIDE 1, CERAMIDE 2, CERAMIDE 3, CERAMIDE 5 and CERAMIDE 6II (all in INCI, 8th Edition) and compounds represented by the following formula are preferred.

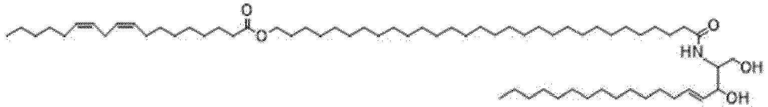

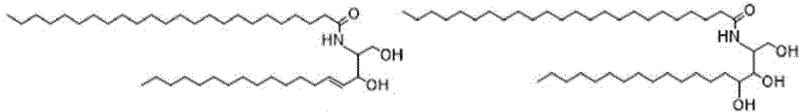

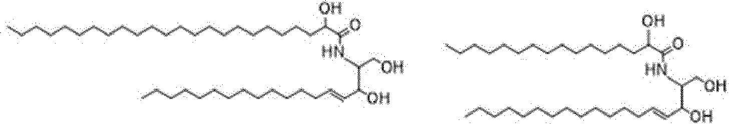

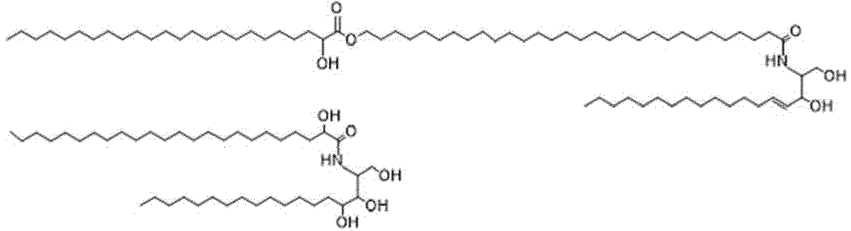

They may be a natural extract or a synthesized product, and a commercially available product may be used. When a commercially available natural ceramide is used, one or more selected from Ceramide I, Ceramide III, Ceramide IIIA, Ceramide IIIB, Ceramide IIIC, Ceramide VI (all from Cosmoferm), Ceramide TIC-001 (Takasago International Corporation), CERAMIDE II (Quest International), DS-Ceramide VI, DS-CLA-Phytoceramide, C6-Phytoceramide, DS-ceramide Y3S (DOOSAN) and CERAMIDE 2 (Sederma) are preferred.

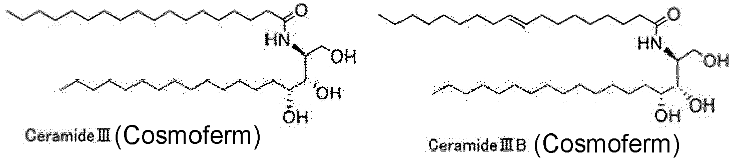

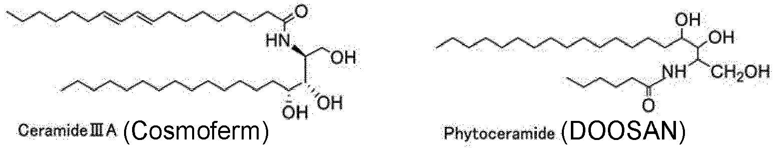

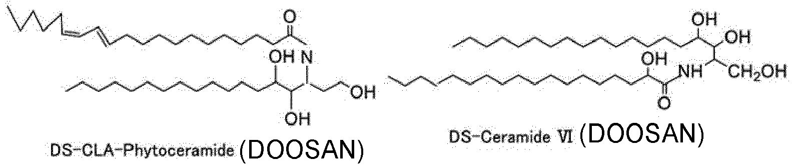

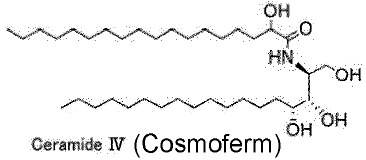

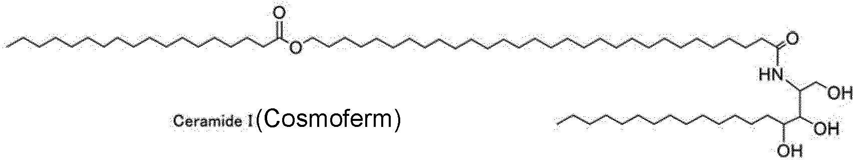

Pseudo-ceramide is represented by the formula (2). 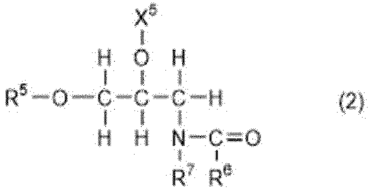 [in which R⁵ represents a linear, branched or cyclic saturated or unsaturated hydrocarbon group having 10 to 22 carbon atoms, which is optionally substituted with a hydroxyl group, or a hydrogen atom; X⁵ represents a hydrogen atom, an acetyl group or a glyceryl group; R⁶ represents a linear, branched or cyclic saturated or unsaturated hydrocarbon group having 5 to 22 carbon atoms, which is optionally substituted with a hydroxyl group or an amino group, or a group in which a linear or branched saturated or unsaturated fatty acid having 8 to 22 carbon atoms, which is optionally substituted with a hydroxyl group, is ester-bonded to the ω terminal of the hydrocarbon group; R⁷ represents a hydrogen atom or an alkyl group having a total of 1 to 30 carbon atoms, which is optionally substituted with a hydroxyl group, a hydroxyalkoxy group, an alkoxy group or an acetoxy group.]

It is particularly preferable that R⁶ is nonyl, tridecyl, pentadecyl, an undecyl group in which linoleic acid is ester-bonded at the ω-position, a pentadecyl group in which linoleic acid is ester-bonded at the ω-position, a pentadecyl group in which 12-hydroxystearic acid is ester-bonded at the ω-position, or an undecyl group in which methyl-branched isostearic acid is amide-bonded at the ω-position.

It is preferable that when R⁵ is a hydrogen atom, R⁷ is an alkyl group having a total of 10 to 30, preferably 12 to 20 carbon atoms, which is optionally substituted with a hydroxyl group, a hydroxyalkoxy group, an alkoxy group or an acetoxy group; and when R⁵ is a linear, branched or cyclic saturated or unsaturated hydrocarbon group having 10 to 22 carbon atoms, which is optionally substituted with a hydroxyl group, R⁷ is a hydrogen atom or an alkyl group having 1 to 8 carbon atoms, which is optionally substituted with a hydroxyl group, a hydroxyalkoxy group, an alkoxy group or an acetoxy group. As the hydroxyalkoxy group or the alkoxy group of R⁷, those having 1 to 7 carbon atoms are preferred.

One or more selected from ceramide of the following formula in which R⁵ is a hexadecyl group, X⁵ is a hydrogen atom, R⁶ is a pentadecyl group and R⁷ is a hydroxyethyl group (i.e., N-(hexadecyloxyhydroxypropyl)-N-hydroxyethylhexadecanamide); and in which R⁵ is a hexadecyl group, X⁵ is a hydrogen atom, R⁶ is a nonyl group and R⁷ is a hydroxyethyl group (i.e., N-(hexadecyloxyhydroxypropyl)-N-hydroxyethyldecanamide) are preferred as the pseudo-ceramide of the formula (2). Among them, the former is particularly preferred.

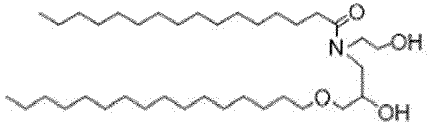

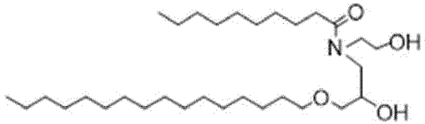

The content of the component (A) in the stock solution is preferably 0.001% by mass or more, more preferably 0.01% by mass or more, further preferably 0.075% by mass or more and further more preferably 0.1% by mass or more, and preferably 15% by mass or less, more preferably 10% by mass or less, further preferably 5.0% by mass or less and further more preferably 3.0% by mass or less from the viewpoint of the improvement of moisturizing effects, emulsion stability and applicability. The content of the component (A) in the stock solution is in a specific range of preferably from 0.001 to 15% by mass, more preferably from 0.010 to 10% by mass, further preferably from 0.075 to 5.0% by mass and further more preferably from 0.1 to 3.0% by mass.

### [Component (B) ionic surfactant]

Any of an anionic surfactant, a cationic surfactant and an amphoteric surfactant may be used as the ionic surfactant of the component (B).

It is preferable that the anionic surfactant contains one or more selected from a carboxylate type such as N-acylamino acid, fatty acid, alkyl ether carboxylic acid, polyoxyethylene alkyl ether carboxylic acid, acyl lactic acid, N-acylmethylalanine, N-acylsarcosine, diacylamino acid and a salt thereof; a sulfonate type such as alkanesulfonic acid, α-olefin sulfonic acid, α-sulfofatty acid methyl ester, acyl isethionic acid, alkyl sulfosuccinic acid, N-acylmethyltaurine and a salt thereof; a sulfate type such as alkyl sulfuric acid ester, polyoxyethylene alkylsulfuric acid ester, alkyl ether sulfuric acid, polyoxyethylene alkyl ether sulfuric acid, fatty acid alkanolamide sulfuric acid ester and a salt thereof; and a phosphate type such as alkyl phosphoric acid ester, polyoxyethylene alkyl ether phosphoric acid and a salt thereof. It is preferable that the anionic surfactant contains one or more selected from N-acylamino acid, fatty acid, alkyl ether carboxylic acid, diacyl glutamate lysine, alkylsulfosuccinic acid, N-acylmethyltaurine, alkyl sulfuric acid ester, polyoxyethylene alkyl sulfuric acid ester, alkyl phosphoric acid ester, polyoxyethylene alkyl ether phosphoric acid and a salt thereof from the viewpoint of emulsion stability.

Of these, it is preferable that the anionic surfactant contains one or more selected from N-acylamino acid, fatty acid, alkyl ether carboxylic acid, diacyl glutamate lysine, alkylsulfosuccinic acid, N-acylmethyltaurine, alkyl sulfuric acid ester, polyoxyethylene alkyl sulfuric acid ester, polyoxyethylene alkyl ether phosphoric acid and a salt thereof. It is more preferable that the anionic surfactant contains one or more selected from N-acylamino acid, fatty acid, alkyl ether carboxylic acid, diacyl glutamate lysine, alkylsulfosuccinic acid, N-acylmethyltaurine, polyoxyethylene alkyl sulfuric acid ester, polyoxyethylene alkyl ether phosphoric acid and a salt thereof. It is further preferable that the anionic surfactant contains one or more selected from N-acylamino acid, fatty acid, diacyl glutamate lysine, N-acylmethyltaurine, polyoxyethylene alkyl ether phosphoric acid and a salt thereof. It is more preferable that the anionic surfactant contains N-acylamino acid and N-acylmethyltaurine, and it is further more preferable that the anionic surfactant contains sodium N-stearoylglutamate and sodium N-stearoyl-N-methyltaurate.

Examples of N-acylmethyltaurine and salts thereof include N-myristoyl-N-methyltaurine, N-lauroyl-N-methyltaurine, N-stearoyl-N-methyltaurine and a salt thereof. Examples of N-acyl amino acids and salts thereof include N-acyl glutamic acid such as N-lauroyl-L-glutamic acid, N-stearoyl-L-glutamic acid and N-myristoyl-L-glutamic acid and a salt thereof. Examples of fatty acids and salts thereof include fatty acids having 12 to 24 carbon atoms such as lauric acid, palmitic acid and stearic acid and a salt thereof. Examples of alkyl ether carboxylic acids and salts thereof include polyoxyethylene lauryl ether acetic acid and a salt thereof. Examples of diacyl glutamate lysine and salts thereof include dilauroyl glutamic acid lysine and a salt thereof. Examples of alkyl sulfosuccinic acid and salts thereof include di-2-ethylhexyl sulfosuccinic acid and a salt thereof. Examples of alkyl sulfuric acid esters and salts thereof include lauryl sulfate and a salt thereof. Examples of polyoxyethylene alkyl sulfuric acid esters and salts thereof include polyoxyethylene lauryl sulfate and a salt thereof. Examples of alkyl phosphoric acid esters and salts thereof include monomyristyl phosphoric acid esters, monostearyl phosphoric acid esters, di (C12-C15) pareth-8-phosphoric acid esters and salts thereof. Examples of polyoxyethylene alkyl ether phosphoric acids and salts thereof include polyoxyethylene oleyl ether phosphoric acid, polyoxyethylene cetyl ether phosphoric acid, polyoxyethylene stearyl ether phosphoric acid and a salt thereof.

Examples of structures of the above salts include alkali metal salts such as sodium salt and potassium salt, basic amino acid salts such as L-arginine salt, L-histidine salt and L-lysine salt, and alkanolamine salts such as triethanolamine salt. These anionic surfactants may be used alone or in combination of two or more.

It is preferable that the cationic surfactant contains one or more selected from a sphingosine salt, an aliphatic amine salt, a quaternary ammonium salt, DL-pyrrolidone carboxylate, alkylamidoamine and a salt thereof, more preferable that the cationic surfactant contains one or more selected from sphingosine salts, mono-C12-24 alkyl trimethyl ammonium salt, di-C12-24 alkyl dimethyl ammonium salt, diacyl ethyl hydroxyethylmonium methosulfate, acyl arginine ethyl pyrrolidone carboxylate and alkylamidoamine salt, and further more preferable that the cationic surfactant contains one or more selected from phytosphingosine glutamate, distearyl dimethylammonium chloride, behenamidopropyl dimethylamine lactate from the viewpoint of the improvement of moisturizing effects and emulsion stability.

Examples of mono-C12-C24 alkyl trimethyl ammonium salts include lauryl trimethyl ammonium chloride, stearyl trimethyl ammonium chloride and behenyl trimethyl ammonium chloride. Examples of di-C12-C24 alkyl dimethyl ammonium salts include dilauryl dimethyl ammonium chloride, distearyl dimethyl ammonium chloride and dibehenyl dimethyl ammonium chloride. Examples of diacylethyl hydroxyethylmonium methosulfate include dicocoyl ethyl hydroxyethylmonium methosulfate. Examples of acyl arginine ethyl pyrrolidone carboxylate include cocoyl arginine ethyl pyrrolidone carboxylate. Examples of alkylamidoamine salts include N-(3-(dimethylamino)propyl)docosanamide (also called behenamidopropyl dimethylamine) and a salt thereof. These cationic surfactants may be used alone or in combination of two or more.

Of the cationic surfactants used in the present invention, sphingosine salts are composed of sphingosine and an acidic substance. Naturally derived sphingosines, synthesized ones with the same structure and a derivative thereof (hereinafter referred to as natural sphingosine) and pseudo-sphingosines with a sphingosine structure (hereinafter referred to as pseudo-sphingosine) are preferred as sphingosine.

Specific examples of natural sphingosines include natural sphingosine, dihydrosphingosine, phytosphingosine, sphingadienine, dehydrosphingosine, dehydrophytosphingosine and an N-alkyl form thereof (e.g., N-methyl form). A natural optically active form (D(+) form), a non-natural optically active form (L(-) form) or a mixture of a natural form and a non-natural form may be used as sphingosine. The relative configuration of the above compounds may be a configuration of the natural form, a configuration of the non-natural form other than that, or a mixture thereof. Furthermore, PHYTOSPHINGOSINE (INCI name, 8th Edition) and those of the following formula are preferred. 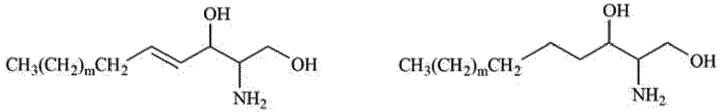 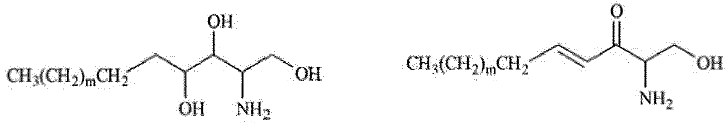 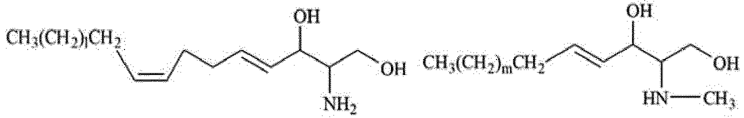 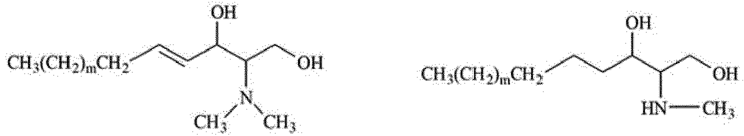 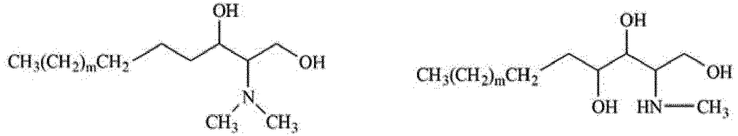 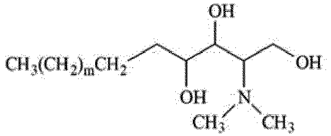 (in the formula, m represents 5 to 7 and I represents 1 to 13)

They may be a natural extract or a synthesized product, and a commercially available product may be used. Examples of commercially available natural sphingosines include D-Sphingosine (4-Sphingenine) (SIGMA-ALDRICH), DS-phytosphingosine (DOOSAN) and phytosphingosine (Cosmoferm).

Examples of pseudo-sphingosines include the following pseudo-sphingosines (i) to (iv) and 1-(2-hydroxyethylamino)-3-isostearyloxy-2-propanol.

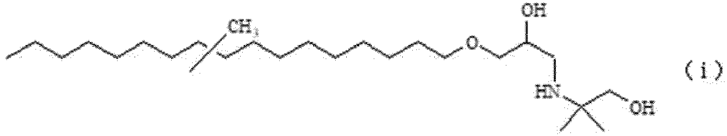

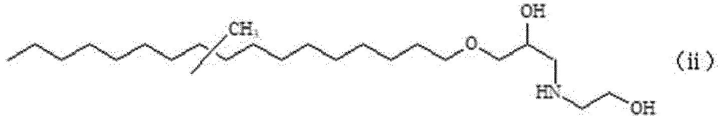

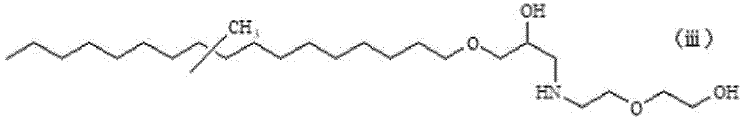

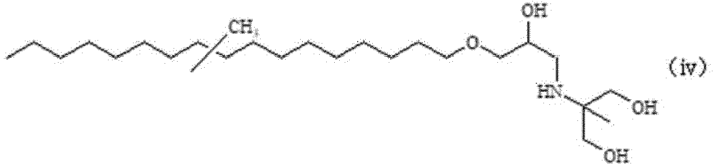

Natural sphingosine, phytosphingosine and pseudo-sphingosine are preferred as sphingosine. Pseudo-sphingosine is more preferred, and pseudo-sphingosine (ii) and 1-(2-hydroxyethylamino)-3-isostearyloxy-2-propanol are further preferred.

Examples of acidic substances which form a salt with the above natural sphingosine, phytosphingosine and pseudo-sphingosine thereof include acidic amino acids such as glutamic acid and aspartic acid; inorganic acids such as phosphoric acid and hydrochloric acid; monocarboxylic acids such as acetic acid; dicarboxylic acid such as succinic acid; and oxycarboxylic acid such as citric acid, lactic acid and malic acid. Of them, salts of natural sphingosine, phytosphingosine and pseudo-sphingosine (pseudo-sphingosine (ii), 1-(2-hydroxyethylamino)-3-isostearyloxy-2-propanol) with succinic acid, lactic acid and glutamic acid are preferred, glutamic acid salts of natural sphingosine, phytosphingosine and pseudo-sphingosine are more preferred, and glutamic acid salts of phytosphingosine are further preferred.

Examples of amphoteric surfactants include phospholipid, alkyldimethylamine oxide, alkylcarboxybetaine, alkylsulfobetaine, amide amino acid salt and alkylamidopropylbetaine. In particular, alkylamidopropylbetaine, soybean phospholipid and hydrogenated soybean phospholipid are preferred, and hydrogenated soybean phospholipid is more preferred.

The content of the component (B) in the stock solution is preferably 0.01% by mass or more, more preferably 0.05% by mass or more, further preferably 0.075% by mass or more, further more preferably 0.15% by mass or more, and preferably 5.0% by mass or less, more preferably 2.0% by mass or less, further preferably 0.75% by mass or less, further more preferably 0.50% by mass or less from the viewpoint of the improvement of moisturizing effects and emulsion stability. The content of the component (B) in the stock solution is in a specific range of preferably from 0.01 to 5.0% by mass, more preferably from 0.05 to 2.0% by mass, further preferably from 0.075 to 0.75% by mass, and further more preferably from 0.15 to 0.50% by mass.

The mass ratio of the component(A) to the component (B), (A)/(B), in the stock solution is preferably 0.003 or more, more preferably 0.03 or more and further preferably 0.3 or more, and preferably 100 or less, more preferably 30 or less and further preferably 15 or less from the viewpoint of the improvement of emulsion stability, moisturizing effects and applicability. The mass ratio of the component (A) to the component (B), (A)/(B), in the stock solution is in a specific range of preferably from 0.003 to 100, more preferably from 0.03 to 30 and further preferably from 0.3 to 15.

### [Component (C): water-soluble polymer]

The water-soluble polymer of the component (C) is not particularly limited as long as it is used for usual external preparation for the skin. Examples thereof include a plant polymer, a microorganism polymer, mucopolysaccharide, a cellulose polymer, a starch polymer, a vinyl polymer, an acrylic polymer and a polyoxyethylene polymer. It is preferable that the water-soluble polymer of the component (C) has a weight average molecular weight of 10,000 or more. The weight average molecular weight is measured by using gel permeation chromatography (GPC) - multiangle laser light scattering detection system (MALLS) with polyethylene oxide as the reference material.

Examples of plant polymers include gum arabic, locust bean gum, guar gum, karaya gum, carrageenan, pectin, agar and starch (rice, corn, potatoes and wheat). Examples of microorganism polymers include xanthan gum and gellan gum. Examples of mucopolysaccharides include hyaluronic acid, tuberose polysaccharide and Tremella fuciformis polysaccharide. Examples of cellulose polymers include methylcellulose, ethylcellulose, carboxymethylcellulose, hydroxymethylcellulose, hydroxyethylcellulose, hydroxypropylcellulose, hydroxypropylmethylcellulose, hydroxypropylmethylcellulose stearoxy ether, nitrocellulose, cellulose sodium sulfate, carboxymethylcellulose sodium salt, crystalline cellulose, cellulose powder and sodium hydroxypropylsulfonate. Examples of starch polymers include carboxymethyl starch and methylhydroxypropyl starch. Examples of vinyl polymers include polyvinylmethyl ether and polyvinyl pyrrolidone. Examples of acrylic polymers include alkyl-modified carboxyvinyl polymer, carboxyvinyl polymer, sodium polyacrylate, polyethyl acrylate, polyacrylic acid amide, acrylic acid/alkyl acrylate copolymer, acrylic acid alkyl methacrylate copolymer, (Sodium acrylate/Sodium acryloyl dimethyltaurine) copolymer, polyacrylate crosspolymer-6, N,N-dimethylaminoethyl methacrylic acid diethyl sulfate · N,N-dimethyl acrylamide · polyethylene glycol dimethacrylate copolymer and 2-methacryloyloxyethyl phosphorylcholine · butyl methacrylate copolymer. Examples of polyoxyethylene polymers include polyethylene oxide. Of them, microorganism polymers, cellulose polymers and acrylic polymers are preferred from the viewpoint of the improvement of emulsion stability and applicability.

Xanthan gum and gellan gum are preferred, and xanthan gum is more preferred as the microorganism polymer. Hydroxypropyl methylcellulose, hydroxyethyl cellulose and hydroxypropyl methylcellulose stearoxy ether are preferred, and hydroxypropyl methylcellulose and hydroxypropyl methylcellulose stearoxy ether are more preferred as the cellulose polymer. Alkyl-modified carboxyvinyl polymer, carboxyvinyl polymer, acrylic acid/ alkyl(meth)acrylate copolymer, N,N-dimethylaminoethyl methacrylic acid diethyl sulfate · N,N-dimethyl acrylamide · polyethylene glycol dimethacrylate copolymer and 2-methacryloyloxyethyl phosphorylcholine · butyl methacrylate copolymer are preferred as the acrylic polymer, and (acrylic acid/ (C10-30) alkyl acrylate) copolymer is more preferred. The (acrylic acid/ (C10-30) alkyl acrylate) copolymer is a copolymer of C10-30 alkyl acrylic acid and acrylic acid, methacrylic acid or a lower alkyl ester thereof, which is cross-linked with allyl ether of sucrose or allyl ether of pentaerythritol. Commercially available products such as Pemulen TR-1, Pemulen TR-2, Carbopol ETD2020, Carbopol 1342 and Carbopol 1382 (all made by Lubrizol Advanced Materials) may be used.

Of them, it is preferable that the component (C) contains one or more selected from hydroxypropyl methylcellulose, hydroxypropyl methylcellulose stearoxy ether, carboxyvinyl polymer, alkyl-modified carboxyvinyl polymer, acrylic acid/ alkyl (meth)acrylate copolymer and 2-methacryloyloxyethyl phosphorylcholine · butyl methacrylate copolymer, and more preferable that the component (C) contains one or more selected from hydroxypropyl methylcellulose, acrylic acid/(C10-30) alkyl acrylate copolymer and 2-methacryloyloxyethyl phosphorylcholine · butyl methacrylate copolymer.

The content of the component (C) in the stock solution is preferably 0.010% by mass or more, more preferably 0.025% by mass or more, further preferably 0.050% by mass or more and further more preferably 0.075% by mass or more, and preferably 2.0% by mass or less, more preferably 0.60% by mass or less, further preferably 0.30% by mass or less and further more preferably 0.20% by mass or less from the viewpoint of the improvement of emulsion stability and applicability. The content of the component (C) in the stock solution is in a specific range of preferably from 0.010 to 2.0% by mass, more preferably from 0.025 to 0.60% by mass, further preferably from 0.050 to 0.30% by mass and further more preferably from 0.075 to 0.20% by mass.

The mass ratio of the component (A) to the component (C), (A)/(C), in the stock solution is preferably 0.01 or more, more preferably 0.10 or more, further preferably 0.50 or more, further preferably 0.75 or more and further preferably 1.0 or more, and preferably 150 or less, more preferably 100 or less, further preferably 40 or less, further preferably 30 or less and further preferably 20 or less from the viewpoint of the improvement of emulsion stability, moisturizing effects and applicability. The mass ratio of the component (A) to the component (C), (A)/(C), in the stock solution is in a specific range of preferably from 0.01 to 150, more preferably from 0.10 to 100, further preferably from 0.50 to 40, further preferably from 0.75 to 30 and further preferably from 1.0 to 20.

The mass ratio of the component (C) to the component (B), (C)/(B), in the stock solution is preferably 0.01 or more, more preferably 0.05 or more, further preferably 0.10 or more and further more preferably 0.20 or more, and preferably 10 or less, more preferably 5 or less, further preferably 1.5 or less and further more preferably 0.8 or less from the viewpoint of the emulsion stability. The mass ratio of the component (C) to the component (B), (C)/(B), in the stock solution is in a specific range of preferably from 0.01 to 10, more preferably from 0.05 to 5, further preferably from 0.10 to 1.5 and further more preferably from 0.20 to 0.8.

### [Component (D): water]

The content of the component (D), water, in the stock solution is preferably 50% by mass or more, more preferably 60% by mass or more, further preferably 70% by mass or more and further more preferably 75% by mass or more, and preferably 98% by mass or less, more preferably 95% by mass or less, further preferably 90% by mass or less and further more preferably 85% by mass or less from the viewpoint of emulsion stability and solubility of carbon dioxide gas. The content of the component (D) in the stock solution is in a specific range of preferably from 50 to 98% by mass, more preferably from 60 to 95% by mass, further preferably from 70 to 90% by mass and further more preferably from 75 to 85% by mass.

### [Nonionic surfactant]

The stock solution may further comprise a nonionic surfactant. Examples of nonionic surfactants include sorbitan fatty acid ester (e.g., monostearic acid sorbitan), glycerol fatty acid ester (e.g., monostearic acid glycerol), polyglycerol fatty acid ester, propylene glycol fatty acid ester, polyethylene glycol fatty acid ester, sucrose fatty acid ester, polyoxyethylene fatty acid ester, polyoxyethylene sorbitan fatty acid ester (e.g., monostearic acid polyoxyethylene (20) sorbitan (polysorbate 60)), polyoxyethylene alkyl ether, alkyl glyceryl ether, polyoxyethylene sorbitol fatty acid ester, polyoxyethylene glycerol fatty acid ester, polyoxyethylene propylene glycol fatty acid ester, polyoxyethylene castor oil, polyoxyethylene hydrogenated castor oil, polyoxyethylene hydrogenated castor oil fatty acid ester, polyoxyethylene phytostanol ether, polyoxyethylene phytosterol ether, polyoxyethylene cholestanol ether, polyoxyethylene cholesteryl ether, polyoxyalkylene-modified organopolysiloxane and polyoxyalkylene · alkyl co-modified organopolysiloxane.

Of them, sorbitan fatty acid ester, glycerol fatty acid ester, alkyl glyceryl ether and polyoxyethylene sorbitan fatty acid ester are preferred, sorbitan C12-22 fatty acid ester, glycerol mono-C12-22 fatty acid ester, mono-C12-22 alkyl glyceryl ether and polyoxyethylene sorbitan C12-22 fatty acid ester are more preferred, and monostearic acid polyoxyethylene sorbitan and monostearic acid sorbitan are more preferred from the viewpoint of the improvement of moisturizing effects and emulsion stability.

### [Other optional components]

The stock solution may also comprise an oil agent which is liquid at 25°C, polyhydric alcohol, a thickener, a preservative, powder, ethanol, an antioxidant, a pigment, a pH adjustor, a perfume, a moisturizing agent, a blood circulation promotor, a cooling agent, an antiperspirant, a fungicide, a whitening gent, an antiinflammatory agent and a skin activator. These agents may be used for a different purpose regardless of the purpose of use of the agent, or may be used for both the original and different purposes depending on what is intended. For example, antiperspirant may be used as perfume, or may be used as an agent which has the effect of both antiperspirant and perfume.

Examples of oil agents which is liquid at 25°C include hydrocarbon oil, silicone oil, ester oil, ether oil and fluorine oil. Hydrocarbon oil, ester oil and silicone oil are preferred, hydrocarbon oil, monoester oil, triester oil and silicone oil are more preferred, and monoester oil, triester oil and silicone oil are further preferred from the viewpoint of the improvement of emulsion stability and applicability. Specific examples thereof include a linear or branched hydrocarbon oil such as light isoparaffin, fluid paraffin, fluid isoparaffin, squalane and squalene; a silicone oil such as cyclomethicone, dimethicone, trisiloxane methyl trimethicone, ethyl trisiloxane, methylphenyl polysiloxane, methyl hydrogen polysiloxane, and higher alcohol modified organopolysiloxane; monoester oil such as isononyl isononanoate, isotridecyl isononanoate and (C12-15) alkyl benzoate, which is an ester of benzoic acid and aliphatic alcohol having 12 to 15 carbon atoms, a diester oil such as neopentylglycol dicaprate, a triester oil such as glyceryl tri(caprylic acid/capric acid) and glyceryl tri(2-ethylhexanoate); an ether oil such as alkyl-1,3-dimethylbutyl ether, dicaprylyl ether and dicaprylyl ether; and a fluorine oil such as fluoropolyether and perfluoroalkyl ether silicone. These oil agents may be derived from a natural substance such as plant. Oil agents which are liquid at 25°C also include liquid ultraviolet absorber.

The content of the oil agent which is liquid at 25°C in the stock solution is preferably 0.01% by mass or more, more preferably 0.05% by mass or more and further preferably 0.1% by mass or more, and preferably 5.0% by mass or less, more preferably 3.0% by mass or less and further preferably 1.0% by mass or less to improve a feeling in use. The content of the oil agent which is liquid at 25°C in the stock solution is in a specific range of preferably from 0.01 to 5.0% by mass, more preferably from 0.05 to 3.0% by mass and further preferably from 0.1 to 1.0% by mass.

Polyhydric alcohol usually used for cosmetics may be used as polyhydric alcohol, and examples thereof include divalent alcohol such as ethylene glycol, propylene glycol, propanediol, 1,3-butylene glycol, 1,3-propanediol, dipropylene glycol, polyethylene glycol and polypropylene glycol; and trivalent or higher valent alcohol such as glycerol and sorbitol. Of them, to improve applicability, divalent alcohol such as propylene glycol, propanediol, 1,3-butyleneglycol, 1,3-propanediol and dipropylene glycol and trivalent or higher valent alcohol such as glycerol is more preferred, and 1,3-propanediol, dipropylene glycol and glycerol are further preferred, and glycerol is further more preferred.

The content of polyhydric alcohol in the stock solution is preferably 1% by mass or more, more preferably 2% by mass or more, further preferably 5% by mass or more, and further more preferably 10% by mass or more, and preferably 50% by mass or less, more preferably 40% by mass or less, further preferably 30% by mass or less and further more preferably 20% by mass or less from the viewpoint of the improvement of moisture retention properties and applicability. The content is preferably from 1 to 50% by mass, more preferably from 2 to 40% by mass, further preferably from 5 to 30% by mass and further more preferably from 10 to 20% by mass.

The stock solution may contain one or more pH adjustors selected from the group consisting of an organic acid, an organic acid salt, an amino acid, an amino acid salt, an amine, an inorganic acid, an inorganic acid salt and an inorganic base. Examples of organic acids include glycolic acid, citric acid, malic acid, succinic acid, tartaric acid and lactic acid. Examples of salts thereof include potassium salts and sodium salts. Examples of amino acids include glutamic acid, aspartic acid, arginine and lysine. Examples of salts thereof include sodium salts, hydrochloride and succinate. Examples of amines include aliphatic amines such as monoethanolamine, triethanolamine, methylamine, ethylamine, trimethylamine, triethylamine, ethylenediamine and tetramethylethylenediamine. Examples of inorganic acids include phosphoric acid, and examples of inorganic acid salts include potassium dihydrogenphosphate and disodium hydrogenphosphate. Examples of inorganic bases include alkali metal hydroxides such as lithium hydroxide, sodium hydroxide and potassium hydroxide. Of them, amino acid is preferred and arginine is more preferred from the viewpoint of moisture retention properties.

### • Propellant

### [Component (E): Carbon dioxide gas]

The present invention comprises the component (E), carbon dioxide gas, as a propellant. Carbon dioxide gas and another propellant may also be used in combination as a propellant. Examples of propellants other than carbon dioxide gas include liquefied petroleum gas (ethane, propane, ethylene, isobutane, n-butane, propylene and a mixed gas thereof (e.g. a mixed gas of isobutane and propane, a mixed gas of propane and butane)), an ether propellant (e.g., dimethyl ether), fluorocarbon (e.g., fluorocarbon, chlorofluorocarbon, bromochlorofluoro carbon), compressed gas (e.g., nitrogen, air, a mixed gas thereof), chlorofluorocarbon gas (e.g., monochlorodifluoroethane, tetrafluoroethane). Of them nitrogen, dimethyl ether and liquefied petroleum gas are preferred. When another propellant is used together with carbon dioxide gas of the component (E), any propellant may be used alone, two or more of them may be used in combination.

When a propellant other than carbon dioxide gas of the component (E) is used in combination as a propellant, the ratio of carbon dioxide gas of the component (E) based on the whole propellant is preferably 20% or more, more preferably 40% or more, further preferably 60% or more and further more preferably 80% or more in terms of the volume of gas (1013.25 hPa, 25°C) so as not to reduce the effect of carbon dioxide gas.

### • Carbonated aerosol external preparation for the skin

The carbonated aerosol external preparation for the skin of the present invention may be produced by preparing a stock solution comprising the components (A) to (D) and packing it in a pressure resistant container together with a propellant comprising the component (E). The type of spray is preferably a foam type that ejects the content in the container in the form of foam.

The ratio of carbon dioxide gas (E) is preferably 0.01 part by mass or more, more preferably 0.10 part by mass or more, further preferably 0.125 part by mass or more, further more preferably 0.25 part by mass or more, yet more preferably 0.5 part by mass or more and yet further preferably 1.0 part by mass or more, and preferably 5.0 parts by mass or less and more preferably 3.0 parts by mass or less based on 100 parts by mass of the stock solution from the viewpoint of improvement of solubility of carbon dioxide gas in the stock solution, the viscosity of foam and spraying properties.

The mass ratio of the component (E) to the component (A), (E)/(A), in the carbonated aerosol external preparation for the skin of the present invention is preferably 0.005 or more, more preferably 0.10 or more and further preferably 0.2 or more, and preferably 3,000 or less, more preferably 300 or less and further preferably 30 or less from the viewpoint of the improvement of emulsion stability of the component (A) and applicability.

The carbonated aerosol external preparation for the skin of the present invention has a pH immediately after discharge of preferably 7.0 or less, more preferably 6.5 or less and further preferably 6.0 or less from the viewpoint of emulsion stability. Furthermore, the carbonated aerosol external preparation for the skin of the present invention has a pH immediately after discharge of preferably 2.0 or more, more preferably 3.0 or more and further preferably 4.0 or more from the viewpoint of skin irritancy. In the present invention, the pH immediately after discharge refers to pH measured 1 minute after discharging the content from the aerosol container at 25°C.

When the carbonated aerosol external preparation for the skin of the present invention is a foam type, the foam discharged has a viscosity of preferably 0.2 Pa·s or more, more preferably 0.5 Pa·s or more, further preferably 2.0 Pa·s or more and further more preferably 5.0 Pa·s or more, and preferably 30 Pa·s or less, more preferably 20 Pa·s or less and further preferably 10 Pa·s or less from the viewpoint of the improvement of applicability.

### [How to use]

The carbonated aerosol external preparation for the skin of the present invention is applied to the skin, more specifically the skin of the whole body excluding the scalp, preferably any one of the face, the body and the limbs, and more preferably the skin of hands and feet in an appropriate amount. The preparation may be dispensed and spread over the hand and applied to the target site, or may be directly discharged to the target site. In the case of a foam type, the preparation may be dispensed and applied to the site so as not to break the foam. It is particularly preferred that the preparation is applied to the site which has thick stratum corneum and can dry up easily, such as hands, feet, fingers, heels, elbows and knees. The amount to be applied is approximately 1 g for both hands.

For the embodiments described above, preferred aspects of the present invention will be described in the following.
<1> A carbonated aerosol external preparation for the skin, comprising:
   a stock solution comprising the following components (A) to (D); and
   a propellant comprising the following component (E):
      (A) ceramide lipid
      (B) an ionic surfactant
      (C) a water-soluble polymer
      (D) water
      (E) carbon dioxide gas.
<2> The carbonated aerosol external preparation for the skin according to <1>, wherein the ceramide lipid of the component (A) preferably comprises one or more selected from a ceramide lipid of the following formula (1) or (2) : 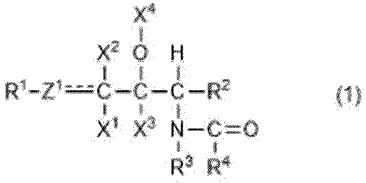 [in which R¹ represents a linear, branched or cyclic saturated or unsaturated hydrocarbon group having 7 to 19 carbon atoms, which is optionally substituted with a hydroxyl group; Z¹ represents a methylene group or a methine group; X¹, X² and X³ are each independently a hydrogen atom, a hydroxyl group or an acetoxy group; X⁴ represents a hydrogen atom or forms an oxo group together with an adjacent oxygen atom (when Z¹ is a methine group, one of X¹ and X² is a hydrogen atom, and the other is not present, and when X⁴ forms an oxo group, X³ is not present); R² represents a hydroxylmethyl group or an acetoxymethyl group; R³ represents a hydrogen atom or an alkyl group having 1 to 4 carbon atoms; R⁴ represents a linear, branched or cyclic saturated or unsaturated hydrocarbon group having 5 to 30 carbon atoms, which is optionally substituted with a hydroxyl group, or a group in which a linear or branched saturated or unsaturated fatty acid having 8 to 22 carbon atoms, which is optionally substituted with a hydroxyl group, is ester-bonded to the ω terminal of the alkyl group; and the dotted line means that the bond may be an unsaturated bond] 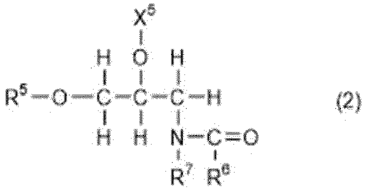 [in which R⁵ represents a linear, branched or cyclic saturated or unsaturated hydrocarbon group having 10 to 22 carbon atoms, which is optionally substituted with a hydroxyl group, or a hydrogen atom; X⁵ represents a hydrogen atom, an acetyl group or a glyceryl group; R⁶ represents a linear, branched or cyclic saturated or unsaturated hydrocarbon group having 5 to 22 carbon atoms, which is optionally substituted with a hydroxyl group or an amino group, or a group in which a linear or branched saturated or unsaturated fatty acid having 8 to 22 carbon atoms, which is optionally substituted with a hydroxyl group, is ester-bonded to the ω terminal of the hydrocarbon group; R⁷ represents a hydrogen atom or an alkyl group having a total of 1 to 30 carbon atoms, which is optionally substituted with a hydroxyl group, a hydroxyalkoxy group, an alkoxy group or an acetoxy group.]
<3> The carbonated aerosol external preparation for the skin according to <1> or <2>, wherein a content of the component (A) in the stock solution is preferably 0.001% by mass or more, more preferably 0.01% by mass or more, further preferably 0.075% by mass or more and further more preferably 0.1% by mass or more, and preferably 15% by mass or less, more preferably 10% by mass or less, further preferably 5.0% by mass or less and further more preferably 3.0% by mass or less.
<4> The carbonated aerosol external preparation for the skin according to any one of <1> to <3>, wherein the component (B) preferably comprises at least one selected from an anionic surfactant selected from N-acylamino acid, fatty acid, alkyl ether carboxylic acid, diacyl glutamate lysine, alkylsulfosuccinic acid, N-acylmethyltaurine, alkyl sulfuric acid ester, polyoxyethylene alkyl sulfuric acid ester, alkyl phosphoric acid ester, polyoxyethylene alkyl ether phosphoric acid and a salt thereof, a cationic surfactant selected from a sphingosine salt, an aliphatic amine salt, a quaternary ammonium salt, DL-pyrrolidone carboxylate, alkylamidoamine and a salt thereof, and an amphoteric surfactant selected from alkylamidopropylbetaine, soybean phospholipid and hydrogenated soybean phospholipid.
<5> The carbonated aerosol external preparation for the skin according to any one of <1> to <4>, wherein a content of the component (B) in the stock solution is preferably 0.01% by mass or more, more preferably 0.05% by mass or more, further preferably 0.075% by mass or more and further preferably 0.15% by mass or more, and preferably 5.0% by mass or less, more preferably 2.0% by mass or less, further preferably 0.75% by mass or less, and further more preferably 0.50% by mass or less.
<6> The carbonated aerosol external preparation for the skin according to any one of <1> to <5>, wherein a mass ratio of the component (A) to the component (B), (A)/(B), in the stock solution is preferably 0.003 or more, more preferably 0.03 or more and further preferably 0.3 or more, and preferably 100 or less, more preferably 30 or less and further preferably 15 or less.
<7> The carbonated aerosol external preparation for the skin according to any one of <1> to <6>, wherein the component (C) comprises preferably one or more selected from a plant polymer, a microorganism polymer, mucopolysaccharide, a cellulose polymer, a starch polymer, a vinyl polymer, an acrylic polymer and a polyoxyethylene polymer, and more preferably one or more selected from hydroxypropyl methylcellulose, hydroxypropyl methylcellulose stearoxy ether, carboxyvinyl polymer, alkyl-modified carboxyvinyl polymer, acrylic acid/ alkyl (meth)acrylate copolymer and 2-methacryloyloxyethyl phosphorylcholine · butyl methacrylate copolymer.
<8> The carbonated aerosol external preparation for the skin according to any one of <1> to <7>, wherein a content of the component (C) in the stock solution is preferably 0.010% by mass or more, more preferably 0.025% by mass or more, further preferably 0.050% by mass or more and further more preferably 0.075% by mass or more, and preferably 2.0% by mass or less, more preferably 0.60% by mass or less, further preferably 0.30% by mass or less and further more preferably 0.20% by mass or less.
<9> The carbonated aerosol external preparation for the skin according to any one of <1> to <8>, wherein a mass ratio of the component (A) to the component (C), (A)/(C), in the stock solution is preferably 0.01 or more, more preferably 0.10 or more, further preferably 0.50 or more, further preferably 0.75 or more and further preferably 1.0 or more, and preferably 150 or less, more preferably 100 or less, further preferably 40 or less, further preferably 30 or less and further preferably 20 or less.
<10> The carbonated aerosol external preparation for the skin according to any one of <1> to <9>, wherein a mass ratio of the component (C) to the component (B), (C)/(B), is preferably 0.01 or more, more preferably 0.05 or more, further preferably 0.10 or more and further more preferably 0.20 or more, and preferably 10 or less, more preferably 5 or less, further preferably 1.5 or less and further more preferably 0.8 or less.
<11> The carbonated aerosol external preparation for the skin according to any one of <1> to <10>, wherein a content of the component (D), water, in the stock solution is preferably 50% by mass or more, more preferably 60% by mass or more, further preferably 70% by mass or more and further more preferably 75% by mass or more, and preferably 98% by mass or less, more preferably 95% by mass or less, further preferably 90% by mass or less and further preferably 85% by mass or less.
<12> The carbonated aerosol external preparation for the skin according to any one of <1> to <11>, wherein the stock solution further comprises polyhydric alcohol.
<13> The carbonated aerosol external preparation for the skin according to <12>, wherein the polyhydric alcohol comprises preferably one or more selected from ethylene glycol, propylene glycol, propanediol, 1,3-butylene glycol, 1,3-propanediol, dipropylene glycol, polyethylene glycol, polypropylene glycol, glycerol and sorbitol, more preferably one or more selected from propylene glycol, propanediol, 1,3-butylene glycol, 1,3-propanediol, dipropylene glycol and glycerol, further more preferably one or more selected from 1,3-propanediol, dipropylene glycol and glycerol and further more preferably glycerol.
<14> The carbonated aerosol external preparation for the skin according to <12> or <13>, wherein a content of polyhydric alcohol in the stock solution is preferably 1% by mass or more, more preferably 2% by mass or more, further preferably 5% by mass or more and further more preferably 10% by mass or more, and preferably 50% by mass or less, more preferably 40% by mass or less, further preferably 30% by mass or less and further more preferably 20% by mass or less.
<15> The carbonated aerosol external preparation for the skin according to any one of <1> to <14>, wherein a mass ratio of the component (E) to the component (A), (E)/(A), is preferably 0.005 or more, more preferably 0.10 or more and further preferably 0.2 or more, and preferably 3,000 or less, more preferably 300 or less and further preferably 30 or less.
<16> The carbonated aerosol external preparation for the skin according to any one of <1> to <15>, wherein a ratio of the component (E) based on 100 parts by mass of the stock solution is preferably 0.01 part by mass or more, more preferably 0.10 part by mass or more, further preferably 0.125 part by mass or more, further more preferably 0.25 part by mass or more, yet more preferably 0.5 part by mass or more and yet further preferably 1.0 part by mass or more, and preferably 5.0 parts by mass or less and more preferably 3.0 parts by mass or less.
<17> The carbonated aerosol external preparation for the skin according to any one of <1> to <16>, wherein the carbonated aerosol external preparation for the skin has a pH immediately after discharge of preferably 7.0 or less, more preferably 6.5 or less and further preferably 6.0 or less, and preferably 2.0 or more, more preferably 3.0 or more and further preferably 4.0 or more.

### Examples

### Examples 1 to 51, Comparative Examples 1 to 5

A stock solution was prepared according to the formulation shown in Tables 1 to 8. The resulting composition was filled in a pressure-resistant container (a 100 mL glass test bottle for aerosol) and the container was sealed, and then pressure was applied to the component (E) to produce an aerosol external preparation for the skin. High-temperature storage stability of the carbonated aerosol external preparations for the skin was evaluated by the following method based on the following criteria.

### <Method for evaluation of high temperature storage stability>

The bottle in which the aerosol had been dissolved was stored in a thermostat bath at 45°C for 30 days, and then the temperature was put back to 25°C. The appearance evaluation and discharge properties were observed based on the following evaluation criteria.

### <Evaluation criteria>

A: No change, dischargeable
B: Slightly viscous but flowable, dischargeable
C: Viscous but flowable, dischargeable
D: Less flowable but no precipitation, dischargeable
E: Non-dischargeable due to precipitation or gelation

### <Method for evaluating viscosity of foam>

Foam was discharged into a 20 mL plastic cup in a full amount and the viscosity was measured by VISCOMETER TVB10 (25°C, rotor No. 3, 12 rpm, 10 s)

### <Measurement of pH immediately after discharge>

The aerosol external preparation for the skin was discharged from the pressure-resistant container under environment of 25°C and approximately 5 g of the preparation was weighed and put into a 20 mL plastic cup. 1 minute after discharge, the pH was measured by a pH meter (LAQUA F-74).

### <Method for evaluating moisturizing effects>

Five expert panelists evaluated moisturizing effects after application when approximately 1 g of the aerosol external preparation for the skin was discharged to the medial side of the upper arm and was spread by the palm, and scored on a scale of highly moist (2 points), moist (1 point) and not moist (0 point). The sum of the evaluation scores is shown in the tables.

### <Method for evaluating applicability>

Five expert panelists evaluated applicability when approximately 1 g of the aerosol external preparation for the skin was discharged to the medial side of the upper arm and was spread by the palm, and scored on a scale of easy to spread (2 points), moderately easy to spread (1 point) and difficult to spread (0 point). The sum of the evaluation scores is shown in the tables.

### <Method for evaluating skin penetration>

Five expert panelists evaluated the penetration of the external preparation into the skin when approximately 1 g of the aerosol external preparation for the skin was discharged to the medial side of the upper arm and was spread by the palm, and scored on a scale of highly penetrating (2 points), moderately penetrating (1 point) and difficult to penetrate (0 point). The sum of the evaluation scores is shown in the tables.

### <Method for evaluating moisture retention effects for extremely dry skin>

Five expert panelists with the skin of a dried shin washed the shin with soap 5 times and approximately 1 g of the aerosol external preparation for the skin was discharged and spread on the skin of the shin. Then the panelists evaluated moist feeling of the skin of the shin after spending 1 hour in a room at 20°C and 40%RH, and scored on a scale of very moist (2 points), moist (1 point) and not moist (0 point). The sum of the evaluation scores is shown in the tables.

**[Table 1]**

| (% by mass; all active amount) | | | Example | | | | | | | | | Comparative Example | | |
|---|---|---|---|---|---|---|---|---|---|---|---|---|---|---|
| | | | 1 | 2 | 3 | 4 | 5 | 6 | 7 | 8 | 9 | 1 | 2 | 3 |
| (A) | N-(hexadecyloxypropyl)-N-hydroxyethylhexadecanamide (*1) | | 1 | 0.001 | 0.01 | 0.075 | 0.1 | 3 | 5 | 10 | 15 | 1 | 1 | 1 |
| (B) | Sodium N-stearoyl-N-methyltaurate (*2) | | 0.33 | 0.33 | 0.33 | 0.33 | 0.33 | 0.33 | 0.33 | 0.33 | 0.33 | 0.33 | - | - |
| (B') | Monostearic acid polyoxyethylene sorbitan (*3) | | - | - | - | - | - | - | - | - | - | - | 0.33 | - |
| | Monostearic acid sorbitan (*4) | | - | - | - | - | - | - | - | - | - | - | - | 0.33 |
| (C) | (Acrylic acid/ (C10-30) alkyl acrylate) copolymer (*5) | | 0.1 | 0.1 | 0.1 | 0.1 | 0.1 | 0.1 | 0.1 | 0.1 | 0.1 | - | 0.1 | 0.1 |
| Others | Glycerol | | 15 | 15 | 15 | 15 | 15 | 15 | 15 | 15 | 15 | 15 | 15 | 15 |
| | Arginine | | 0.15 | 0.15 | 0.15 | 0.15 | 0.15 | 0.15 | 0.15 | 0.15 | 0.15 | - | 0.15 | 0.15 |
| (D) | Purified water | | Balance | Balance | Balance | Balance | Balance | Balance | Balance | Balance | Balance | Balance | Balance | Balance |
| Total amount of stock solution | | | 100 | 100 | 100 | 100 | 100 | 100 | 100 | 100 | 100 | 100 | 100 | 100 |
| Stock solution: (E) carbon dioxide gas | | | 100:2.2 | 100:2.2 | 100:2.2 | 100:2.2 | 100:2.2 | 100:2.2 | 100:2.2 | 100:2.2 | 100:2.2 | 100:2.2 | 100:2.2 | 100:2.2 |
| Mass ratio (A)/(B) | | | 3.0 | 0.003 | 0.03 | 0.2 | 0.3 | 9.1 | 15.2 | 30.3 | 45.5 | 3.0 | - | - |
| Mass ratio (A)/(C) | | | 10 | 0.01 | 0.1 | 0.75 | 1 | 30 | 50 | 100 | 150 | - | 10 | 10 |
| Mass ratio (C)/(B) | | | 0.3 | 0.3 | 0.3 | 0.3 | 0.3 | 0.3 | 0.3 | 0.3 | 0.3 | 0 | - | - |
| Mass ratio (E)/(A) | | | 2.2 | 2200 | 220 | 29 | 22 | 0.73 | 0.44 | 0.22 | 0.14 | 2.2 | 2.2 | 2.2 |
| pH immediately after discharge | | | 5.8 | 5.9 | 5.9 | 5.8 | 5.8 | 5.8 | 5.7 | 5.6 | 5.6 | 5.5 | 5.7 | 5.7 |
| Viscosity of foam(Pa·s) | | | 6.1 | 0.3 | 0.5 | 2 | 2.2 | 8.9 | 10 | 22 | 30 | 0.2 | 2.2 | 3.5 |
| Evaluation | | High temperature storage stability(45°C, 1 month) | A | A | A | A | A | A | B | C | D | E | E | E |
| | | Moisturizing effect | 10 | 5 | 7 | 8 | 9 | 10 | 10 | 10 | 10 | 7 | 8 | 8 |
| | | Applicability | 10 | 3 | 5 | 7 | 8 | 10 | 10 | 8 | 6 | 3 | 7 | 7 |

**[Table 2]**

| (% by mass; all active amount) | | | Example | | | | | | | |
|---|---|---|---|---|---|---|---|---|---|---|
| | | | 10 | 11 | 12 | 13 | 14 | 15 | 16 | 17 |
| (A) | N-(hexadecyloxypropyl)-N-hydroxyethylhexadecanamide (*1) | | 1 | 1 | 1 | 1 | 1 | 1 | 1 | 1 |
| (B) | Sodium N-stearoyl-N-methyltaurate (*2) | | 0.33 | 0.33 | 0.33 | 0.33 | 0.33 | 0.33 | 0.33 | 0.33 |
| (C) | Acrylic acid/ (C10-30) alkyl acrylate copolymer (*5) | | 0.01 | 0.025 | 0.05 | 0.075 | 0.2 | 0.3 | 0.6 | 2 |
| Others | Glycerol | | 15 | 15 | 15 | 15 | 15 | 15 | 15 | 15 |
| | Arginine | | 0.015 | 0.0375 | 0.075 | 0.1125 | 0.3 | 0.45 | 0.9 | 3 |
| (D) | Purified water | | Balanc e | Balanc e | Balanc e | Balanc e | Balanc e | Balanc e | Balanc e | Balance |
| Total amount of stock solution | | | 100 | 100 | 100 | 100 | 100 | 100 | 100 | 100 |
| Stock solution: (E) carbon dioxide gas | | | 100:2.2 | 100:2.2 | 100:2.2 | 100:2.2 | 100:2.2 | 100:2.2 | 100:2.2 | 100:2.2 |
| Mass ratio (A)/(B) | | | 3.0 | 3.0 | 3.0 | 3.0 | 3.0 | 3.0 | 3.0 | 3.0 |
| Mass ratio (A)/(C) | | | 100 | 40 | 20 | 13.3 | 5.0 | 3.3 | 1.7 | 0.5 |
| Mass ratio (C)/(B) | | | 0.03 | 0.075 | 0.15 | 0.23 | 0.61 | 0.91 | 1.82 | 6.1 |
| Mass ratio (E)/(A) | | | 2.2 | 2.2 | 2.2 | 2.2 | 2.2 | 2.2 | 2.2 | 2.2 |
| pH immediately after discharge | | | 5.6 | 5.7 | 5.8 | 5.8 | 5.7 | 5.7 | 5.5 | 5.5 |
| Viscosity of foam(Pa·s) | | | 2.1 | 3.5 | 4.6 | 5.9 | 10 | 10 | 10 | 10 |
| Evaluation | | High temperature storage stability(45°C, 1 month) | D | C | B | A | A | B | C | D |
| | | Moisturizing effect | 9 | 9 | 10 | 10 | 10 | 10 | 10 | 10 |
| | | Applicability | 7 | 8 | 8 | 10 | 10 | 10 | 10 | 9 |

**[Table 3]**

| (% by mass) | | Example | | | | | | | | |
|---|---|---|---|---|---|---|---|---|---|---|
| | | 18 | 19 | 20 | 21 | 22 | 23 | 24 | 25 | 26 |
| (A) | N-(hexadecyloxypropyl)-N-hydroxyethylhexadecanamide (*1) | 1 | 1 | 1 | 1 | 1 | 1 | 1 | 1 | 1 |
| (B) | Sodium N-stearoyl-N-methyltaurate (*2) | 0.01 | 0.05 | 0.075 | 0.15 | 0.2 | 0.5 | 0.75 | 2 | 5 |
| (C) | Acrylic acid/ (C10-30) alkyl acrylate copolymer (*5) | 0.1 | 0.1 | 0.1 | 0.1 | 0.1 | 0.1 | 0.1 | 0.1 | 0.1 |
| Others | Glycerol | 15 | 15 | 15 | 15 | 15 | 15 | 15 | 15 | 15 |
| | Arginine | 0.15 | 0.15 | 0.15 | 0.15 | 0.15 | 0.15 | 0.15 | 0.15 | 0.15 |
| (D) | Purified water | Balance | Balance | Balance | Balance | Balance | Balance | Balance | Balance | Balance |
| Total amount of stock solution | | 100 | 100 | 100 | 100 | 100 | 100 | 100 | 100 | 100 |
| Stock solution: (E) carbon dioxide gas | | 100:2.2 | 100:2.2 | 100:2.2 | 100:2.2 | 100:2.2 | 100:2.2 | 100:2.2 | 100:2.2 | 100:2.2 |
| Mass ratio (A)/(B) | | 100.0 | 20.0 | 13.3 | 6.7 | 5.0 | 2.0 | 1.3 | 0.5 | 0.2 |
| Mass ratio (A)/(C) | | 10.0 | 10.0 | 10.0 | 10.0 | 10.0 | 10.0 | 10.0 | 10.0 | 10.0 |
| Mass ratio (C)/(B) | | 10 | 2 | 1.3 | 0.67 | 0.5 | 0.2 | 0.13 | 0.05 | 0.02 |
| Mass ratio (E)/(A) | | 2.2 | 2.2 | 2.2 | 2.2 | 2.2 | 2.2 | 2.2 | 2.2 | 2.2 |
| pH immediately after discharge | | 5.8 | 5.8 | 5.8 | 5.8 | 5.8 | 5.8 | 5.8 | 5.9 | 6.0 |
| Viscosity of foam(Pa·s) | | 1.5 | 2.5 | 3.2 | 4.2 | 5.1 | 6.3 | 6.5 | 7.5 | 7.6 |
| Evaluation | High temperature storage stability(45°C, 1 month) | D | C | B | A | A | A | B | C | D |
| | Moisturizing effect | 9 | 9 | 10 | 10 | 10 | 10 | 10 | 10 | 10 |
| | Applicability | 6 | 7 | 7 | 8 | 9 | 10 | 10 | 10 | 10 |

**[Table 4]**

| (% by mass; all active amount) | | Example | | | |
|---|---|---|---|---|---|
| | | 27 | 28 | 29 | 30 |
| (A) | N-(hexadecyloxypropyl)-N-hydroxyethylhexadecanamide (*1) | 1 | 1 | 1 | 1 |
| (B) | Sodium N-stearoyl-N-methyltaurate (*2) | 0.15 | 0.15 | 0.75 | 0.75 |
| (C) | Acrylic acid/ (C10-30) alkyl acrylate copolymer (*5) | 0.05 | 0.6 | 0.05 | 0.6 |
| Others | Glycerol | 15 | 15 | 15 | 15 |
| | Arginine | 0.075 | 0.9 | 0.075 | 0.9 |
| (D) | Purified water | Balance | Balance | Balance | Balance |
| Total amount of stock solution | | 100 | 100 | 100 | 100 |
| Stock solution: (E) carbon dioxide gas | | 100:2.2 | 100:2.2 | 100:2.2 | 100:2.2 |
| Mass ratio (A)/(B) | | 6.7 | 6.7 | 1.3 | 1.3 |
| Mass ratio (A)/(C) | | 20.0 | 1.7 | 20.0 | 1.7 |
| Mass ratio (C)/(B) | | 0.33 | 4 | 0.07 | 0.8 |
| Mass ratio (E)/(A) | | 2.2 | 2.2 | 2.2 | 2.2 |
| pH immediately after discharge | | 5.7 | 5.7 | 5.8 | 5.8 |
| Viscosity of foam(Pa·s) | | 4 | 10 | 4.5 | 10 |
| Evaluation | High temperature storage stability(45°C, 1 month) | A | C | C | A |
| | Moisturizing effect | 10 | 10 | 10 | 10 |
| | Applicability | 7 | 10 | 7 | 10 |

**[Table 5]**

| (% by mass; all active amount) | | Example | | | | | | |
|---|---|---|---|---|---|---|---|---|
| | | 31 | 32 | 33 | 34 | 35 | 36 | 37 |
| (A) | N-(hexadecyloxypropyl)-N-hydroxyethylhexadecanamide (*1) | 1 | 1 | 1 | 1 | 1 | 1 | 1 |
| (B) | Sodium N-stearoyl-N-methyltaurate (*2) | 0.33 | 0.33 | 0.33 | 0.33 | 0.33 | 0.33 | 0.33 |
| (C) | Acrylic acid/ (C10-30) alkyl acrylate copolymer (*5) | - | - | - | - | - | - | 0.1 |
| | Hydroxypropyl methylcellulose stearoxy ether (*6) | 0.1 | - | - | - | - | - | - |
| | Carboxyvinyl polymer (*7) | - | 0.1 | - | - | - | - | - |
| | Solution of 2-methacryloyloxyethyl phosphorylcholine · butyl methacrylate copolymer (*8) | - | - | 0.1 | - | - | - | - |
| | Hydroxypropyl methylcellulose (*9) | - | - | - | 0.1 | - | - | 0.1 |
| | Xanthan gum (*10) | - | - | - | - | 0.1 | - | - |
| | N,N-dimethylaminoethyl methacrylic acid diethyl sulfate · N,N-dimethyl acrylamide · polyethylene glycol dimethacrylate copolymer (*1 1) | - | - | - | - | - | 0.1 | - |
| Others | Glycerol | 15 | 15 | 15 | 15 | 15 | 15 | 15 |
| | Arginine | - | 0.15 | - | - | - | 0.15 | 0.15 |
| (D) | Purified water | Balance | Balanc e | Balance | Balance | Balanc e | Balanc e | Balance |
| Total amount of stock solution | | 100 | 100 | 100 | 100 | 100 | 100 | 100 |
| Stock solution: (E) carbon dioxide gas | | 100:2.2 | 100:2.2 | 100:2.2 | 100:2.2 | 100:2.2 | 100:2.2 | 100:2.2 |
| Mass ratio (A)/(B) | | 3.0 | 3.0 | 3.0 | 3.0 | 3.0 | 3.0 | 3.0 |
| Mass ratio (A)/(C) | | 10.0 | 10.0 | 10.0 | 10.0 | 10.0 | 10.0 | 5.0 |
| Mass ratio (C)/(B) | | 0.3 | 0.3 | 0.3 | 0.3 | 0.3 | 0.3 | 0.3 |
| Mass ratio (E)/(A) | | 2.2 | 2.2 | 2.2 | 2.2 | 2.2 | 2.2 | 2.2 |
| pH immediately after discharge | | 5.9 | 5.7 | 5.9 | 5.8 | 5.9 | 5.9 | 5.8 |
| Viscosity of foam(Pa·s) | | 9.5 | 5.1 | 6 | 8 | 3 | 2.1 | 7.8 |
| Evaluatio n | High temperature storage stability(45°C, 1 month) | A | A | A | A | A | A | A |
| | Moisturizing effect | 10 | 9 | 10 | 10 | 8 | 8 | 10 |
| | Applicability | 9 | 9 | 10 | 10 | 7 | 7 | 10 |

**[Table 6]**

| (% by mass; all active amount) | | Example | | | | | |
|---|---|---|---|---|---|---|---|
| | | 38 | 39 | 40 | 41 | 42 | 43 |
| (A) | N-(hexadecyloxypropyl)-N-hydroxyethylhexadecanamide (*1) | 1 | 1 | 1 | 1 | 1 | 1 |
| (B) | Phytosphingosine glutamic acid salt (*12) | 0.33 | - | - | - | - | - |
| | Distearyl dimethylammonium chloride (*13) | - | 0.33 | - | - | - | - |
| | Behenamidopropyl dimethylamine lactate (*14) | - | - | 0.33 | - | - | - |
| | Sodium N-stearoylglutamate (*15) | - | - | - | 0.33 | - | - |
| | Sodium N-stearoyl-N-methyltaurate | - | - | - | - | - | 0.33 |
| | Hydrogenated soybean phospholipid (*16) | - | - | - | - | 0.33 | - |
| (B') | Monostearic acid polyoxyethylene sorbitan (*3) | | - | - | - | - | 0.1 |
| (C) | Acrylic acid/ (C10-30) alkyl acrylate copolymer (*5) | 0.1 | 0.1 | 0.1 | 0.1 | 0.1 | 0.1 |
| Others | Glycerol | 15 | 15 | 15 | 15 | 15 | 15 |
| | Arginine | 0.15 | 0.15 | 0.15 | 0.15 | 0.15 | 0.15 |
| (D) | Purified water | Balance | Balance | Balance | Balance | Balance | Balance |
| Total amount of stock solution | | 100 | 100 | 100 | 100 | 100 | 100 |
| Stock solution: (E) carbon dioxide gas | | 100:2.2 | 100:2.2 | 100:2.2 | 100:2.2 | 100:2.2 | 100:2.2 |
| Mass ratio (A)/(B) | | 3.0 | 3.0 | 3.0 | 3.0 | 3.0 | 3.0 |
| Mass ratio (A)/(C) | | 10.0 | 10.0 | 10.0 | 10.0 | 10.0 | 10.0 |
| Mass ratio (C)/(B) | | 0.3 | 0.3 | 0.3 | 0.3 | 0.3 | 0.3 |
| Mass ratio (E)/(A) | | 2.2 | 2.2 | 2.2 | 2.2 | 2.2 | 2.2 |
| pH immediately after discharge | | 5.5 | 5.4 | 5.3 | 5.9 | 5.9 | 5.8 |
| Viscosity of foam(Pa·s) | | 6.4 | 7.1 | 6.5 | 6.6 | 4.8 | 6.7 |
| Evaluation | High temperature storage stability(45°C, 1 month) | A | A | A | A | A | A |
| | Moisturizing effect | 10 | 10 | 10 | 10 | 8 | 10 |
| | Applicability | 10 | 10 | 10 | 10 | 8 | 10 |

**[Table 7]**

| (% by mass; all active amount) | | Example | | | | | | |
|---|---|---|---|---|---|---|---|---|
| | | 44 | 45 | 46 | 47 | 48 | 49 | 50 |
| (A) | N-(hexadecyloxypropyl)-N-hydroxyethylhexadecanamide (*1) | 1 | 1 | 1 | 1 | 1 | 1 | 1 |
| (B) | Sodium N-stearoyl-N-methyltaurate (*2) | 0.33 | 0.33 | 0.33 | 0.33 | 0.33 | 0.33 | 0.33 |
| (C) | Acrylic acid/ (C10-30) alkyl acrylate copolymer (*5) | 0.1 | 0.1 | 0.1 | 0.1 | 0.1 | 0.1 | 0.1 |
| Others | Glycerol | 15 | 15 | 15 | 15 | 15 | 15 | 15 |
| | Arginine | 0.15 | 0.15 | 0.15 | 0.15 | 0.15 | 0.15 | 0.15 |
| (D) | Purified water | Balance | Balance | Balance | Balance | Balance | Balance | Balance |
| Total amount of stock solution | | 100 | 100 | 100 | 100 | 100 | 100 | 100 |
| Stock solution: (E) carbon dioxide gas | | 100:0.01 | 100:0.10 | 100:0.125 | 100:0.25 | 100:0.5 | 100:1.0 | 100:2.5 |
| Mass ratio (A)/(B) | | 3.0 | 3.0 | 3.0 | 3.0 | 3.0 | 3.0 | 3.0 |
| Mass ratio (A)/(C) | | 10.0 | 10.0 | 10.0 | 10.0 | 10.0 | 10.0 | 10.0 |
| Mass ratio (C)/(B) | | 0.3 | 0.3 | 0.3 | 0.3 | 0.3 | 0.3 | 0.3 |
| Mass ratio (E)/(A) | | 0.01 | 0.1 | 0.125 | 0.25 | 0.5 | 1 | 2.5 |
| pH immediately after discharge | | 6.4 | 6.2 | 6.0 | 5.9 | 5.8 | 5.8 | 5.6 |
| Viscosity of foam(Pa·s) | | 0.3 | 0.8 | 2 | 2.5 | 5 | 6.3 | 9.5 |
| Evaluation | High temperature storage stability(45°C, 1 month) | A | A | A | A | A | A | A |
| | Moisturizing effect | 4 | 5 | 6 | 8 | 9 | 10 | 10 |
| | Applicability | 3 | 5 | 7 | 8 | 9 | 10 | 10 |

**[Table 8]**

| (% by mass; all active amount) | | Example | | Comparative Example | |
|---|---|---|---|---|---|
| | | 1 | 51 | 4 | 5 |
| (A) | N-(hexadecyloxypropyl)-N-hydroxyethylhexadecanamide (*1) | 1 | 1 | 1 | 1 |
| (B) | Sodium N-stearoyl-N-methyltaurate (*2) | 0.33 | 0.33 | 0.33 | 0.33 |
| (C) | Acrylic acid/ (C10-30) alkyl acrylate copolymer (*5) | 0.1 | 0.1 | 0.1 | 0.1 |
| Others | Glycerol | 15 | 15 | 15 | 15 |
| | Arginine | 0.15 | 0.1 | 0.1 | 0.1 |
| (D) | Purified water | Balance | Balance | Balance | Balance |
| Total amount of stock solution | | 100 | 100 | 100 | 100 |
| Stock solution: (E) carbon dioxide gas | | 100:2.2 | 100:0.01 | 100:0 | - |
| Stock solution: LPG | | - | - | - | 95:5 |
| Mass ratio (A)/(B) | | 3.0 | 3.0 | 3.0 | 3.0 |
| Mass ratio (A)/(C) | | 10 | 10 | 10 | 10 |
| Mass ratio (C)/(B) | | 0.3 | 0.3 | 0.3 | 0.3 |
| Mass ratio (E)/(A) | | 2.2 | 0.01 | - | - |
| pH immediately after discharge | | 5.8 | 5.7 | 5.7 | 5.8 |
| Viscosity of foam(Pa·s) | | 6.1 | 0.3 | - | 15 |
| Evaluation | High temperature storage stability (45°C, 1 month) | A | A | A | C |
| | Moisturizing effect | 10 | 4 | 3 | 3 |
| | Applicability | 10 | 3 | 2 | 3 |
| | Moisture retention effects for extremely dry skin | 8 | 4 | 2 | 4 |
| | Penetration | 10 | 5 | 2 | 3 |

*1: Sphingolipid E (made by Kao Corporation)
*2: Nikkol SMT (made by Nikko Chemicals Co., Ltd.)
*3: RHEODOL TW-S120V (made by Kao Corporation)
*4: RHEODOL SP-S10V (made by Kao Corporation)
*5: PEMULEN TR1 (made by Lubrizol Advanced Materials)
*6: SANGELOSE 90L (made by Daido Chemical Corporation)
*7: Carbopol 980 (made by Lubrizol Advanced Materials)
*8: LIPIDURE-PMB (made by NOF Corporation)
*9: METOLOSE 90SH15000 (made by Shin-Etsu Chemical Co., Ltd.)
*10: KELDENT (made by DSP Gokyo Food & Chemical)
*11: Polymer KG30 (made by Kao Corporation)
*12: Phytosphingosine (made by Evonik Nutrition & Care)
*13: VARISOFT TA 100 (made by Evonik Nutrition & Care)
*14: NIKKOL Amidoamine MPB PLUS (made by Nikko Chemicals Co., Ltd.)
*15: Amisoft HS-11P (Ajinomoto Co., Inc.)
*16: COATSOME NC-21 (made by NOF Corporation)

## Claims

1. A carbonated aerosol external preparation for the skin, comprising:
a stock solution comprising the following components (A) to (D); and
a propellant comprising the following component (E):
(A) ceramide lipid
(B) an ionic surfactant
(C) a water-soluble polymer
(D) water
(E) carbon dioxide gas.

2. The carbonated aerosol external preparation for the skin according to claim 1, wherein a mass ratio of the component (C) to the component (B), (C)/(B), is from 0.01 to 10.

3. The carbonated aerosol external preparation for the skin according to claim 1 or 2, wherein a content of the component (A) in the stock solution is from 0.001 to 15% by mass.

4. The carbonated aerosol external preparation for the skin according to any one of claims 1 to 3, wherein a content of the component (B) in the stock solution is from 0.01 to 5.0% by mass.

5. The carbonated aerosol external preparation for the skin according to any one of claims 1 to 4, wherein a content of the component (C) in the stock solution is from 0.010 to 2.0% by mass.

6. The carbonated aerosol external preparation for the skin according to any one of claims 1 to 5, wherein the component (C) comprises one or more selected from hydroxypropyl methylcellulose, hydroxypropyl methylcellulose stearoxy ether, carboxyvinyl polymer, alkyl-modified carboxyvinyl polymer, acrylic acid/ alkyl (meth)acrylate copolymer and 2-methacryloyloxyethyl phosphorylcholine · butyl methacrylate copolymer.

7. The carbonated aerosol skin external preparation for the skin according to any one of claims 1 to 6, wherein a mass ratio of the component (E) to the component (A), (E)/(A), is from 0.005 to 3,000.

8. The carbonated aerosol skin external preparation for the skin according to any one of claims 1 to 7, wherein a ratio of the component (E) based on 100 parts by mass of the stock solution is from 0.01 to 5.0 parts by mass.
